# EUROPÄISCHE PATENTSCHRIFT

(11) **EP 2 817 094 B1**
(45) Veröffentlichungstag und Bekanntmachung des Hinweises auf die Patenterteilung: **10.04.2019**
(21) Anmeldenummer: 13705170.2
(22) Anmeldetag: 20.02.2013
(51) Int. Cl.: B01J 37/02, B01J 37/16, B01J 37/18, B01J 23/52, B01J 23/58, B01J 23/66, B01J 35/00, C07C 67/055

(54) **VORVERGOLDUNG VON PD-AU-GECOATETEN SCHALENKATALYSATOREN**
PRE-GOLD-PLATING PD-AU-COATED SHELL CATALYSTS
PRÉDORURE DE CATALYSEURS ENROBÉS REVÊTUS DE PD-AU

(30) Priorität: 20.02.2012 DE 102012003236
(43) Veröffentlichungstag der Anmeldung: 31.12.2014
(73) Patentinhaber: Clariant Produkte (Deutschland) GmbH, 65929 Frankfurt am Main (DE)
(72) Erfinder: HAGEMEYER, Alfred, Sunnyvale, California 94086 (US); FISCHER, Carolin, 83024 Rosenheim (DE); MESTL, Gerhard, 80935 München (DE); SCHECK, Peter, 82205 Gilching (DE); BOBKA, Roman, 81377 München (DE)
(74) Vertreter: Clariant Produkte (Deutschland) GmbH
(86) Internationale Anmeldenummer: PCT/EP2013/053334
(87) Internationale Veröffentlichungsnummer: WO 2013/124293

(56) Entgegenhaltungen:
- WO-A2-2008/145395
- US-A- 5 691 267
- US-A- 5 693 586
- US-A1- 2010 197 488

## Beschreibung

Die vorliegende Erfindung betrifft ein neues Verfahren zur Herstellung eines Schalenkatalysators, der zur Herstellung von Vinylacetatmonomer (VAM) geeignet ist. Die vorliegende Erfindung betrifft zudem einen Schalenkatalysator, der durch das erfindungsgemäße Verfahren erhältlich ist sowie die Verwendung des erfindungsgemäßen Schalenkatalysators zur Herstellung von VAM.

Geträgerte Katalysatoren, die Palladium und Gold enthalten, sind bereits seit einiger Zeit bekannt. VAM wird üblicherweise in Gegenwart von Katalysatoren enthaltend Palladium und Gold aus einem Reaktionsgemisch aus Ethylen, Sauerstoff und Essigsäure hergestellt. Diverse Herstellungsverfahren solcher geträgerten Katalysatoren sind bereits bekannt. So werden beispielsweise Vorläuferverbindungen, die die entsprechenden Metalle enthalten, gelöst in vorzugsweise einer wässrigen Lösung auf die Oberfläche eines Trägerkörpers aufgetragen. Der die entsprechenden Vorläuferverbindungen enthaltende Trägerkörper wird dann üblicherweise unter oxidierenden Bedingungen in einem Hochtemperaturofen kalziniert, wobei die metallhaltigen Vorläuferverbindungen in die Metalloxide überführt werden. Anschließend werden die Trägerkörper, die die entsprechenden Metalloxide enthalten, dann der Reduktion zu den elementaren Metallen unterzogen.

Vinylacetatmonomer ist ein wichtiger Baustein für die Herstellung von Polyvinylacetat, von Vinylacetat-Copolymeren (wie Ethylenvinylacetaten oder Ethylen-Vinylalkohol-Copolymeren) und von Polyvinylalkohol. Aufgrund des weiten Anwendungsgebiets dieser Polymere, bspw. als Bindemittel im Bau-, Farben-, und Lacksektor und als Rohstoff für die Klebstoff-, Papier- und Textilindustrie besteht nach wie vor ein hoher Bedarf an VAM und an der ständigen Verbesserung der Aktivität und Selektivität von Katalysatoren zu dessen Herstellung.

Normalerweise werden bei der Synthese von VAM Schalenkatalysatoren eingesetzt, bei denen sich in einer äußeren Schale eines Katalysatorträgerkörpers elementares Pd und Au befindet. Zu deren Herstellung wird in der Regel eine Mischlösung aus einer Pd-haltigen Vorläuferverbindung und einer Au-haltigen Vorläuferverbindung auf einen Katalysatorträgerkörper aufgebracht, dieser anschließend getrocknet und die Metallkomponenten der Vorläuferverbindungen in die elementaren Metalle übergeführt.

So offenbart die US 2010/0197488 A1 die Aufbringung einer Mischlösung aus Pd- und Au-haltigen Vorläuferverbindungen auf einen Katalysatorträgerkörper mittels Imprägnierung, während dieser in Form einer Schüttung in einem Fließbett vorliegt.

WO 2008/145395 A2 beschreibt ebenfalls die Aufbringung einer Mischlösung aus Pd- und Au-haltigen Vorläuferverbindungen auf einen Katalysatorträgerkörper mittels Imprägnierung, während dieser in Form einer Schüttung in einem Fließbett vorliegt. Außerdem wird der Katalysatorträger zusätzlich dotiert.

In US 5,693,586 wird der Katalysatorträger durch Imprägnierung einer Mischlösung aus Pd- und Au-haltigen Vorläuferverbindungen behandelt, eine Schüttung in Form eines Fließbetts liegt dabei nicht vor.

Die Kombination Pd/Au führt in der Regel zu einer guten Selektivität bzw. Aktivität des Katalysators. Wegen der Kapitalintensität entsprechender VAM-Produktionsanlagen und der zunehmend hohen Rohstoffpreise, insbesondere für Ethlyen, besteht jedoch ein stetiges Bedürfnis, die Wirtschaftlichkeit des Verfahrens zur Herstellung von VAM durch verbesserte Katalysatoren zu optimieren.

Es war deshalb die Aufgabe der vorliegenden Erfindung ein Verfahren zur Herstellung eines Schalenkatalysators bereitzustellen, das zu Schalenkatalysatoren führt, die hinsichtlich der Aktivität und Selektivität in der Synthese von VAM bisherige Katalysatoren übertreffen.

Diese Aufgabe wurde durch ein erfindungsgemäßes Verfahren gelöst, mit dem Schalenkatalysatoren mit signifikant erhöhter Selektivität und Aktivität für VAM hergestellt werden können. Das erfindungsgemäße Verfahren zur Herstellung eines Schalenkatalysators ist durch die folgenden Verfahrensschritte gekennzeichnet:
(a) Unterziehen einer Schüttung eines Katalysatorträgerkörpers (Trägerkörper) einer Umwälzbewegung;
(b) In-Kontaktbringen einer zerstäubten, wässrigen Lösung enthaltend eine Au-haltige Vorläuferverbindung mit der der Umwälzbewegung unterzogenen Schüttung des Katalysatorträgerkörpers durch Aufsprühen;
(c) In-Kontaktbringen einer zerstäubten, wässrigen Lösung enthaltend eine Pd-haltige Vorläuferverbindung mit dem nach Schritt (b) erhaltenen Katalysatorträgerkörper; und
(d) Reduktion der Metallkomponenten der Vorläuferverbindungen zu den elementaren Metallen durch Unterziehen des in Schritt (c) erhaltenen Katalysatorträgerkörpers einer Temperaturbehandlung in einer nicht-oxidierenden Atmosphäre,
worin der Schalenkatalysator einen Anteil an Au im Bereich von 0,1 bis 1,0 Gew.-%, bezogen auf das Gesamtgewicht des Schalenkatalysators, und einen Anteil an Pd im Bereich von 0,6 bis 2,0 Gew.-%, bezogen auf das Gesamtgewicht des Schalenkatalysators, aufweist.

In Schritt (c) des erfindungsgemäßen Verfahrens ist es ebenso bevorzugt, dass das In-Kontaktbringen stattfindet, indem die Lösung auf eine einer Umwälzbewegung unterzogenen Schüttung des Katalysatorträgerkörpers aufgesprüht wird.

Gemäß einer Ausführungsform des erfindungsgemäßen Verfahrens werden in Schritt (b) neben den katalytischen Au-Vorläuferverbindungen keine weiteren Vorläuferverbindungen katalytisch aktiver Metalle, insbesondere keine Pd-Vorläuferverbindungen aufgebracht.

Unter dem Begriff "Schalenkatalysator" versteht man einen Katalysator, der einen Trägerkörper und eine Schale mit katalytisch-aktivem Material umfasst, wobei die Schale auf zwei verschiedene Arten gebildet werden kann:
Zum Einen kann ein katalytisch-aktives Material in dem äußeren Bereich des Trägerkörpers vorliegen, sodass das Material des Trägerkörpers als Matrix für das katalytisch-aktive Material dient und der Bereich des Trägerkörpers, der mit dem katalytisch-aktiven Material imprägniert ist, eine Schale um den nicht imprägnierten Kern des Trägerkörpers bildet. Zum Anderen kann auf die Oberfläche des Trägerkörpers eine zusätzliche Schicht aufgebracht sein, in der ein katalytisch-aktives Material vorliegt. Diese Schicht bildet somit eine zusätzliche Materiallage, die als Schale um den Trägerkörper aufgebaut wird. In der letzteren Variante ist das Trägerkörpermaterial kein Bestandteil der Schale, sondern die Schale wird durch das katalytisch-aktive Material selbst oder einem Matrixmaterial, das ein katalytisch-aktives Material umfasst, gebildet. In einer Ausführungsform der vorliegenden Erfindung handelt es sich vorzugsweise um die zuerst genannte Variante eines Schalenkatalysators.

In dem nach dem erfindungsgemäßen Verfahren hergestellten Katalysator liegen die Metalle entweder monoatomar oder in der Form von Aggregaten vor. Vorzugsweise liegen sie jedoch in der Form von Aggregaten vor. Diese monoatomaren Atome oder Aggregate sind innerhalb der Schale des Schalenkatalysators überwiegend gleichmäßig dispergiert. Unter einem Aggregat versteht man die Zusammenlagerung mehrerer Metallatome zu einem Verbund, der zwischen monoatomarer Form und metallischem Erscheinungsbild liegt. Darunter fallen auch sogenannte Metallcluster.

Die Schalendicke der äußeren Schale des Trägerkörpers beträgt vorzugsweise 1 bis 70 %, stärker bevorzugt 2 bis 60%, noch stärker bevorzugt 3 bis 50 % und am stärksten bevorzugt 4 bis 40 % der Hälfte der Gesamtdicke des Trägerkörpers. Die genannte Prozentzahl bezieht sich deshalb auf die Hälfte der Gesamtdicke, da je nach Form des Trägerkörpers bei der Herstellung, z. B. durch Sprühimprägnierung mit einer Vorläuferverbindung enthaltenden Lösung, die Vorläuferverbindung entweder von zwei äußeren Oberflächen in das Trägerkörpermaterial eindringt (Kugel), oder wenn das Trägerkörpermaterial eine komplexere Form, wie z. B. die eines Hohlzylinders, aufweist, es eine äußere Oberfläche und eine innere Oberfläche gibt, in die die Vorläuferverbindung eindringt. Bei von der Kugelgeometrie abweichenden Trägerkörpermaterialien wird die Gesamtdicke des Trägers entlang der längsten Trägerkörperachse gemessen. Die äußere Schalengrenze wird mit der äußeren Grenze des metallhaltigen Trägerkörpers gleichgesetzt. Unter innerer Schalengrenze versteht man die im Inneren des Trägerkörpers befindliche Grenze der äußeren metallhaltigen Schale, die soweit von der äußeren Schalengrenze entfernt ist, dass sich 95 Gew.-% des gesamten im Trägerkörper enthaltenden Metalls in der äußeren Schale befindet. Hierbei ist die Schalendicke jedoch vorzugsweise nicht größer als 70 %, bevorzugt nicht größer als 60 %, stärker bevorzugt nicht größer als 50 %, noch stärker bevorzugt nicht größer als 40 % und am stärksten bevorzugt nicht größer als 30 %, jeweils bezogen auf die Hälfte der Gesamtdicke des Trägerkörpers.

Vorzugsweise enthält der metallimprägnierte Trägerkörper in seinem inneren Bereich, also innerhalb des Bereichs, der nach außen durch die innere Schalengrenze der Metallschale begrenzt ist, nicht mehr als 5 % des gesamten Metalls.

Im Hinblick auf die Schalendicke des Katalysators liegt die maximale Konzentration an Metall vorzugsweise im Bereich der äußeren Schale, besonders bevorzugt am äußeren Rand der äußeren Schale, also der geometrischen Katalysatoroberfläche nahe gelegen. Die Metallkonzentration nimmt vorzugsweise in Richtung der inneren Schalengrenze ab.

Der Trägerkörper ist vorzugsweise aus einem inerten Material. Er kann porös oder nicht porös sein. Vorzugsweise ist der Trägerkörper jedoch porös. Der Trägerkörper besteht vorzugsweise aus Teilchen mit einer regulären oder irregulären Form, wie beispielsweise Kugeln, Tabletten, Zylindern, Vollzylindern oder Hohlzylindern, Ringen, Sternen oder anderen Formen, und weist in seinen Dimensionen, wie z. B. Durchmesser, Länge oder Breite einen Bereich von 1 bis 10 mm, bevorzugt 3 bis 9 mm auf. Sphärische, d. h. z. B. kugelförmige Teilchen mit einem Durchmesser von 3 bis 8 mm sind erfindungsgemäß bevorzugt. Das Trägerkörpermaterial kann jegliche nicht poröse und poröse Substanz, vorzugsweise poröse Substanz umfassen. Beispiele für Materialen hierfür sind Titanoxid, Siliciumoxid, Aluminiumoxid, Zirkoniumoxid, Magnesiumoxid, Siliciumcarbid, Magnesiumsilicat, Zinkoxid, Zeolithe, Schichtsilikate und Nanomaterialen, wie beispielsweise Kohlenstoffnanotubes oder Kohlenstoffnanofasern, vorzugsweise dann, wenn das Trägerkörpermaterial selbst ein heterogener Katalysator ist.

Die vorgenannten oxidischen Trägerkörpermaterialen können beispielsweise in Form von Mischoxiden oder definierten Zusammensetzungen eingesetzt werden, wie beispielsweise TiO₂, SiO₂, Al₂O₃, ZrO₂, MgO, SiC oder ZnO. Weiterhin können vorzugsweise Ruße, Ethylenschwarz, Kohle, Graphit, Hydrotalcite oder weitere dem Fachmann an sich bekannte Trägerkörpermaterialen in verschiedenen möglichen Modifikationen eingesetzt werden. Die Trägerkörpermaterialen können vorzugsweise etwa mit Alkali- oder Erdalkalimetallen oder auch mit Phosphor-, Halogenid- und/oder Sulfatsalzen dotiert sein. Insbesondere bevorzugt ist es, wenn der Katalysatorträger SiO₂ umfasst, vorzugsweise in einer Menge im Bereich von 65 bis 98 Gew.-%, bezogen auf das Gesamtgewicht des Trägerkörpers. Die oxidischen Trägerkörpermaterialien können auch einen Anteil an Zirkoniumdioxid aufweisen. Der Anteil an ZrO₂ an diesem Materialien liegt hierbei vorzugsweise im Bereich von 5 bis 20 Gew.-%.

Die BET-Oberfläche des Trägerkörpermaterials ohne die Beschichtung mit den Vorläuferverbindungen beträgt 1 bis 1.000 m²/g, vorzugsweise 10 bis 600 m²/g, besonders bevorzugt 20 bis 400 m²/g und ganz besonders bevorzugt zwischen 100 und 200 m²/g. Die BET-Oberfläche wird nach der 1-Punkt-Methode durch Absorption von Stickstoff nach DIN 66 132 bestimmt.

Darüber hinaus kann es bevorzugt sein, dass das integrale Porenvolumen des Trägerkörpermaterials (bestimmt gemäß DIN 66133 (Hg-Porosimetrie)) ohne die Beschichtung mit der Vorläuferverbindung größer als 0,1 ml/g ist, vorzugsweise größer als 0,18 ml/g und am bevorzugtesten im Bereich von 0,25 bis 0,5 ml/g.

Das Schüttgewicht des Trägerkörpers liegt vorzugsweise im Bereich von 480 bis 650 g/l, stärker bevorzugt im Bereich von 500 bis 630 g/l.

Der Trägerkörper wird üblicherweise hergestellt, indem eine Vielzahl von Trägerkörpern einem "Batch"-Verfahren unterworfen werden, bei dessen einzelnen Verfahrensschritten die Formkörper beispielsweise durch Verwendung von Rühr- und Mischwerkzeugen verhältnismäßig hohen mechanischen Belastungen unterliegen.

Darüber hinaus kann der durch das erfindungsgemäße Verfahren hergestellte Schalenkatalysator beim Befüllen eines Reaktors mechanisch stark beansprucht werden, wodurch es zu einer unerwünschten Staubentwicklung sowie einer Beschädigung des Trägerkörpers, insbesondere seiner in einem äußeren Bereich gelegenen, katalytisch-aktiven Schale kommen kann.

Insbesondere um den Abrieb des durch das erfindungsgemäße Verfahren hergestellten Katalysators in vertretbaren Grenzen zu halten, weist der Schalenkatalysator eine Härte von größer/gleich 20 N auf, vorzugsweise eine von größer/gleich 25 N, weiter bevorzugt eine von größer/gleich 35 N und noch stärker bevorzugt eine von größer/gleich 40 N, insbesondere bevorzugt im Bereich von 40 bis 65 N. Die Ermittlung der Härte ist dabei mittels eines Tablettenhärtetesters 8 M der Firma Dr. Schleuniger Pharmathron AG an 99 Stück Schalenkatalysatoren als Durchschnitt bestimmt, nach Trocknung des Katalysators bei 130 °C für zwei Stunden, wobei die Geräteeinstellungen wie folgt sind:
Distanz zum Formkörper: 5,00 mm
Zeitverzögerung: 0,80 s
Vorschub-Typ: 6 D
Geschwindigkeit: 0,60 mm pro Sekunde

Die Härte des durch das erfindungsgemäße Verfahren hergestellten Katalysators kann beispielsweise mittels Variationen gewisser Parameter des Verfahrens zur Herstellung des Trägerkörpers beeinflusst werden, beispielsweise durch die Kalzinierungsdauer und/oder die Kalzinierungstemperatur des Trägerkörpers. Bei der soeben genannten Kalzinierung handelt es sich nicht um eine Kalzinierung des mit den metallhaltigen Vorläuferverbindungen imprägnierten Trägerkörpers, sondern lediglich um einen Kalzinierschritt zur Herstellung des Trägerkörpers bevor die Vorläuferverbindungen aufgetragen werden.

Es ist zudem bevorzugt, dass 80 % des integralen Porenvolumens des Trägerkörpers von Mesoporen und Makroporen gebildet sind, vorzugsweise mindestens 85 % und am stärksten bevorzugt mindestens 90 %. Dadurch wird einer durch Diffusionslimitierung bewirkten verminderten Aktivität des durch das erfindungsgemäße Verfahren hergestellten Katalysators entgegengewirkt, insbesondere bei metallhaltigen Schalen mit verhältnismäßig großen Dicken. Dabei sollen diesbezüglich unter den Begriffen Mikroporen, Mesoporen und Makroporen Poren verstanden werden, die einen Durchmesser von kleiner als 2 nm, einen Durchmesser von 2 bis 50 nm bzw. einen Durchmesser von größer als 50 nm aufweisen.

Die Aktivität der durch das erfindungsgemäße Verfahren hergestellten Schalenkatalysatoren hängt in der Regel von der Menge der Metallbeladung in der Schale ab: In der Regel ist sie je höher, desto mehr Metall sich in der Schale befindet. Die Dicke der Schale hat hier einen geringeren Einfluss auf die Aktivität, ist jedoch eine entscheidende Größe hinsichtlich der Selektivität der Katalysatoren. Die Selektivität der durch das erfindungsgemäße Verfahren hergestellten Schalenkatalysatoren ist im Allgemeinen, bei gleicher Metallbeladung des Katalysatorträgers, je höher, desto kleiner die Dicke der äußeren Schale des Katalysators ist. Es ist also entscheidend, ein optimales Verhältnis von Metallbeladung zu Schalendicke einzustellen, um eine möglichst hohe Selektivität bei möglichst hoher Aktivität zu gewährleisten. Entsprechend einer weiter bevorzugten Ausführungsform des durch das erfindungsgemäße Verfahren hergestellten Katalysators weist daher die Schale des Katalysators eine Dicke im Bereich von 5 µm bis 2000 µm, bevorzugt eine von 10 µm bis 1500 µm, stärker bevorzugt eine von 15 bis 1000 µm auf. Soll der Schalenkatalysator beispielsweise als Katalysator für die Vinylacetatsynthese eingesetzt werden, liegt seine Schalendicke vorzugsweise im Bereich von 10 µm bis 400 µm, stärker bevorzugt im Bereich von 15 µm bis 300 µm.

Die Dicke der Schale kann mittels eines Mikroskops optisch ausgemessen werden. Und zwar erscheint der Bereich, in dem das Metall abgeschieden wird, schwarz, während die Edelmetallfreien Bereiche weiß erscheinen. Die Grenzlinie zwischen Edelmetall-haltigen und -freien Bereichen ist in der Regel sehr scharf und optisch deutlich zu erkennen. Sollte die vorgenannte Grenzlinie nicht scharf ausgebildet und entsprechend optisch nicht deutlich zu erkennen sein, so entspricht die Dicke der Schale - wie bereits erwähnt - der Dicke einer Schale, gemessen ausgehend von der äußeren Oberfläche des Katalysatorträgers, in der 95 % des auf dem Träger abgeschiedenen Edelmetalls enthalten ist.
Um eine weitgehend einheitliche Aktivität des durch das erfindungsgemäße Verfahren hergestellten Katalysators über die Dicke der edelmetallhaltigen Schale hinweg zu gewährleisten, sollte die Edelmetallkonzentration über die Schalendicke hinweg nur verhältnismäßig wenig variieren. Bevorzugt ist es daher, wenn das Profil der Edelmetallkonzentration des Katalysators über einen Bereich von 90 % der Schalendicke hinweg, wobei der Bereich zur äußeren und inneren Schalengrenze jeweils um 5 % der Schalendicke beabstandet ist, von der mittleren Edelmetallkonzentration dieses Bereichs um maximal +/- 20 % abweicht, vorzugsweise um maximal +/- 15 und bevorzugt um maximal +/- 10 %. Derartige Profile sind mittels des weiter unten beschriebenen In-Kontaktbringens einer zerstäubten, wässrigen Lösung enthaltend die Vorläuferverbind(en) auf eine einer Umwälzbewegung unterliegenden Schüttung eines Katalysatorträgerkörpers durch Aufsprühen erhältlich. Um die Umwälzbewegung der Schüttung der Trägerkörper zu erreichen, ist es insbesondere geeignet, die Schüttung der Träger in einer Wirbelschicht, einem Fließbett oder einem Innojet-Aircoater wie weiter unten beschrieben vorzulegen. Die soeben genannte Verteilung der Metallbeladung beschreibt vorzugsweise eine Rechteckfunktion. Neben der Rechteckfunktion kann die Metallbeladung innerhalb Schale aber auch eine Dreieck- oder Trapezfunktion beschreiben, bei der die Metallkonzentration graduell von außen nach innen in der Schale abfällt. Es ist deshalb erfindungsgemäß bevorzugt, dass jegliches in dieser Anmeldung genannte Aufbringen von Vorläuferverbindungen auf die oben genannte Art durchgeführt.

Es ist zudem in dem erfindungsgemäßen Verfahren möglich, dass während dem Schritt (c), d.h. dem Aufsprühen (Beschichten) der Pd-haltigen Vorläuferverbindung auch eine Mischlösung aus einer Pd-haltigen Vorläuferverbindung und einer Au-haltigen Vorläuferverbindung aufgesprüht wird, d.h. die in Schritt (c) aufgesprühte Lösung kann zusätzlich noch eine Au-haltige Vorläuferverbindung enthalten. Alternativ dazu kann auch während dem Aufsprühen der Pd-haltigen Vorläuferverbindung aus einer Lösung zusätzlich eine Au-haltige Vorläuferverbindung aus einer getrennten Lösung aufgesprüht werden. Wird während dem Schritt (c) auch eine Au-haltige Vorläuferverbindung aufgesprüht, ist es bevorzugt, dass diese aus einer getrennten Lösung von der Pd-haltigen Vorläuferverbindung aufgebracht wird, insbesondere deshalb, damit das Aufsprühen der Au-haltigen Vorläuferverbindung des Schrittes (b) während des Schritts (c) dem Aufsprühen der Pd-haltigen Vorläuferverbindung fortgeführt werden kann. In anderen Worten wird vorzugsweise zunächst nur eine Lösung enthaltend eine Au-haltige Vorläuferverbindung aufgesprüht. Nach einer bestimmten Aufsprühzeit der Au-haltigen Vorläuferverbindung wird vorzugsweise zusätzlich eine Lösung enthaltend eine Pd-haltige Vorläuferverbindung aufgesprüht. Das Aufsprühen der Au-haltigen Vorläuferverbindung kann fortgesetzt werden bis zum Ende des Aufsprühens der Pd-haltigen Vorläuferverbindung, kann jedoch auch vor dem Ende des Aufsprühens der Pd-haltigen Vorläuferverbindung enden.

Weiterhin bevorzugt ist es, dass nach dem Schritt (c) weiterhin eine Lösung enthaltend eine Au-haltige Vorläuferverbindung auf den Trägerkörper aufgesprüht wird. Dieser optionale Schritt wird in dieser Anmeldung als Schritt (c1) bezeichnet und wird vorzugsweise vor dem Schritt (d) durchgeführt.

Das erfindungsgemäße Verfahren kann folglich in den folgenden Varianten durchgeführt werden: Schritt (b): Aufsprühen der Lösung enthaltend die Au-haltige Vorläuferverbindung; Schritt (c): Aufsprühen der Lösung enthaltend die Pd-haltige Vorläuferverbindung; das Aufsprühen einer Au-haltigen Vorläuferverbindung kann während des Schritts (c) optional auch erfolgen, bzw. das Aufsprühen der Lösung enthaltend die Au-haltige Vorläuferverbindung kann während des Schritts (c) ganz oder teilweise fortgesetzt werden; optionaler Schritt (c1): hier kann weiterhin eine Lösung enthaltend eine Au-haltige Vorläuferverbindung aufgesprüht werden; der Schritt (c1) kann unabhängig davon erfolgen, dass in Schritt (c) zusätzlich eine Au-haltige Vorläuferverbindung aufgesprüht wird. Wird jedoch im Schritt (c) die ganze Zeit ebenso eine Au-haltige Vorläuferverbindung aufgesprüht, so ist es bevorzugt, dass das Aufsprühen der Au-haltigen Vorläuferverbindung einfach ohne Unterbrechung in Schritt (c1) fortgesetzt wird. In Schritt (c1) wird jedoch vorzugsweise nur eine Au-haltige Vorläuferverbindung und keine Pd-haltige Vorläuferverbindung aufgesprüht.

Unabhängig davon, welche der genannten Verfahrensvarianten gewählt wird, ist für den Erhalt eines Schalenkatalysators mit erhöhter Selektivität und Aktivität in erster Linie entscheidend, dass das Aufsprühen der Au-haltigen Vorläuferverbindung vor dem Aufsprühen der Pd-haltigen Vorläuferverbindung durchgeführt wird. D.h. während des Schritts (a) des Aufsprühens der Lösung enthaltend die Au-haltige Vorläuferverbindung erfolgt kein zusätzliches Aufsprühen einer Pd-haltigen Vorläuferverbindung. Das schließt jedoch nicht aus, dass während des Schritts des Aufsprühens einer Lösung enthaltend die Pd-haltige Vorläuferverbindung auch eine Au-haltige Vorläuferverbindung aufgesprüht wird.

Das Aufsprühen der Vorläuferverbindungen in den Schritten (b) und (c) des erfindungsgemäßen Verfahrens auf den Trägerkörper lässt sich nach an sich bekannten Verfahren durchführen.

Im Stand der Technik erfolgt der Auftrag einer die Vorläuferverbindungen enthaltenden Lösung oft durch Tränkung, indem der Trägerkörper in die Vorläuferverbindungs-Lösungen eingetaucht wird, oder gemäß dem Incipient-Wetness-Verfahren. Mit Hilfe dieser Tränkungsverfahren ist es jedoch schwierig, einen Schalenkatalysator mit einer definierten Schale und einer homogenen Metallverteilung herzustellen.

Das Aufsprühen der Vorläuferverbindungen in den Schritten (b) und (c) und dem optionalen Schritt (c1) in dem erfindungsgemäßen Verfahren wird vorzugsweise durchgeführt, indem der Trägerkörper mit einer die Vorläuferverbindung enthaltenden wässrigen Lösung besprüht wird. Dabei wird eine Schüttung des Trägerkörpers einer Umwälzbewegung unterzogen, so dass der Trägerkörper von allen Seiten gleichmäßig besprüht werden kann. Die Umwälzbewegung kann prinzipiell durch jegliches bekannte mechanische Rührgerät, wie beispielsweise eine Dragiertrommel erfolgen. Es ist jedoch erfindungsgemäß besonders bevorzugt, dass die Umwälzbewegung der Trägerkörper mithilfe eines Prozessgases durchgeführt wird, z. B. in einem Fließbett, einer Wirbelschicht oder in einer Beschichtungskammer eines Innojet-Aircoaters. Dabei werden die Trägerkörper bewegt, indem das Prozessgas eingeblasen wird. Das Prozessgas wird hier vorzugsweise so geführt, dass die Trägerkörper in einer kontrollierten Gleitschicht des Prozessgases gehalten werden. Das Prozessgas wird hier vorzugsweise erwärmt, sodass das Lösungsmittel rasch verdampft. Auf diese Weise liegen die Vorläuferverbindungen in der genannten definierten Schale des Trägerkörpers vor. Die Sprührate wird während des Aufsprühens vorzugsweise so gewählt, dass ein Gleichgewicht zwischen der Abdampfrate des Lösungsmittel und der Zuführungsrate der Vorläuferverbindungen auf dem Trägerkörper erreicht wird. Dies ermöglicht, die gewünschte Schalendicke und Palladium/Goldverteilung in der Schale einzustellen. Je nach Sprührate kann somit die Schalendicke stufenlos eingestellt und optimiert werden, beispielsweise bis zu einer Dicke von 2 mm. Aber auch sehr dünne Schalen mit einer Dicke von kleiner als 1000 µm sind so möglich.

Es ist besonders bevorzugt, dass die Sprührate bei dem Aufsprühen in allen denkbaren Schritten des Aufbringens von Vorläuferverbindungen konstant ist und im Bereich eines Massenstroms der Lösung enthaltend die Vorläuferverbindung(en) von 0,5 bis 10 g/min pro 100 g zu beschichtendem Trägerformkörper, stärker bevorzugt im Bereich von 1 bis 8 g/min pro 100 g zu beschichtendem Trägerkörper, noch stärker bevorzugt 2 bis 6 g/min pro 100 g zu beschichtendem Trägerkörper und am stärksten bevorzugt 3,5 bis 5 g/min zu beschichtendem Trägerkörper liegt. In anderen Worten liegt das Verhältnis des Gewichts der aufgesprühten Lösung zu dem Gewicht der Schüttung des Trägerkörpers im Bereich von 0,005 bis 0,1, stärker bevorzugt 0,01 bis 0,08, noch stärker bevorzugt 0,02 bis 0,06 und am stärksten bevorzugt 0,035 bis 0,05. Ein Massenstrom oder Verhältnis oberhalb des angegebenen Bereiches führt zu Katalysatoren mit geringerer Selektivität, ein Massenstrom oder Verhältnis unterhalb des angegebenen Bereichs hat zwar keine stark negativen Auswirkungen auf die Katalysatorperformance, jedoch ist der Zeitaufwand der Katalysatorherstellung sehr hoch und die Herstellung damit uneffizient.

Ausgehend von einer konstanten Sprührate bei dem Aufbringen aller Lösungen, ist es bevorzugt, wenn die Zeit des Aufbringens der Lösung in Schritt (b) zu der Zeit des Aufbringens der Lösung in Schritt (c) in einem Verhältnis im Bereich von 5:1 zu 1:5, bevorzugt 4:1 zu 1:2, stärker bevorzugt 3:1 zu 1:1 und am stärksten bevorzugt 2,5:1 zu 1,5:1 liegt.

Wird eine Fließbett-Anlage verwendet, ist es bevorzugt, wenn die Trägerkörper in dem Fließbett elliptisch oder toroidal umlaufen. Um eine Vorstellung davon zu geben, wie sich die Trägerkörper in derartigen Fließbetten bewegen, sei ausgeführt, dass bei "elliptischem Umlauf" sich die Trägerkörper in dem Fließbett in vertikaler Ebene auf einer elliptischen Bahn bewegen mit wechselnder Größe der Haupt-und Nebenachse. Bei "toroidalem" Umlauf bewegen sich die Trägerkörper in dem Fließbett in vertikaler Ebene auf einer elliptischen Bahn mit wechselnder Größe der Haupt- und Nebenachse und in horizontaler Ebene auf einer Kreisbahn mit wechselnder Größe des Radius. Im Mittel bewegen sich die Trägerkörper bei "elliptischem Umlauf" in vertikaler Ebene auf einer elliptischen Bahn, bei "toroidalem Umlauf" auf einer toroidalen Bahn, d. h., dass ein Trägerkörper die Oberfläche eines Torus mit vertikal elliptischem Schnitt helikal abfährt.

Das Aufsprühen der Vorläuferverbindungen auf den Trägerkörper in den Schritten (b) und (c) und dem optionalen Schritt (c1) des erfindungsgemäßen Verfahrens wird besonders bevorzugt mittels eines Fließbetts in einer Fließbettanlage durchgeführt. Besonders bevorzugt ist es dabei, dass in der Anlage eine sogenannte kontrollierte Gleitschicht aus Prozessgas besteht. Zum Einen werden die Trägerkörper durch die kontrollierte Gleitschicht aus Prozessgas durchmischt, wobei sie gleichzeitig um ihre eigene Achse rotieren und dabei gleichmäßig von dem Prozessgas getrocknet werden. Zum Anderen passieren die Trägerkörper aufgrund der durch die kontrollierte Gleitschicht aus Prozessgas bewirkten konsequenten Orbitalbewegung der Trägerkörper den Sprühvorgang (Applikation der Vorläuferverbindungen) in nahezu konstanter Häufigkeit. Dadurch wird eine weitgehend einheitliche Schalendicke, bzw. Eindringtiefe der Edelmetalle in den Trägerkörper, einer behandelten Phase von Trägerkörpern erreicht. Ferner wird dadurch erreicht, dass die Edelmetallkonzentration über einen verhältnismäßig großen Bereich der Schalendicke hinweg nur verhältnismäßig gering variiert, d. h., dass die Edelmetallkonzentration über einen großen Bereich der Schalendicke hinweg in etwa eine Rechteckfunktion beschreibt, wodurch eine weitgehend einheitliche Aktivität des resultierenden Katalysators über die Dicke der Edelmetall-Schale hinweg gewährleistet ist. Es ist auf diese Weise aber auch möglich, die Edelmetallkonzentration in der Schale so einzustellen, dass diese eine Dreieck- oder Trapezfunktion beschreibt.

Geeignete konventionelle Dragiertrommeln, Wirbelschichtanlagen bzw. Fließbettanlagen zur Durchführung des Aufsprühens der Vorläuferverbindungen im erfindungsgemäßen Verfahren sind im Stand der Technik bekannt und werden zum Beispiel von Unternehmen wie Heinrich Brucks GmbH (Alfeld, Deutschland), ERWEKA GmbH (Heusenstamm, Deutschland), Stechel (Deutschland), DRIAM Anlagenbau GmbH (Erichskirch, Deutschland), Glatt GmbH (Binzen, Deutschland), D.S. Divisione Verniciatura (Osteria, Italien), HOFER-Pharma Maschinen GmbH (Weil am Rhein, Deutschland), L.B. Bohle Maschinen und Verfahren GmbH (Enningerloh, Deutschland), Lödige Maschinenbau GmbH (Paderborn, Deutschland), Manesty (Merseyside, Großbritannien), Vector Corporation (Marion (IA) USA), Aeromatic-Fielder AG (Bubendorf, Schweiz), GEA Process Engineering (Hampshire, Großbritannien), Fluid Air Inc. (Aurora, Illinois, USA), Heinen Systems GmbH (Varel, Deutschland), Hüttlin GmbH (Steinen, Deutschland), Umang Pharmatech Pvt. Ltd. (Maharaschtra, Indien) und Innojet Technologies (Lörrach, Deutschland) vertrieben. Besonders bevorzugte Fließbettvorrichtungen werden mit der Bezeichnung Innojet® Aircoater oder Innojet® Ventilus von der Firma Innojet Technologies vertrieben. Insbesondere bevorzugt wird hier der Coater IAC-5, der Coater IAC-150 oder der Coater IAC-025 jeweils der Firma Innojet verwendet.

Weiterhin wird der in dem erfindungsgemäßen Verfahren eingesetzte Trägerkörper während des Aufsprühens der die Vorläuferverbindungen enthaltenden Lösungen in den Schritten (b) und (c) und dem optionalen Schritt (c1) erwärmt, beispielsweise mittels erwärmtem Prozessgas. Das Prozessgas weist hier vorzugsweise eine Temperatur von 10 bis 110 °C, stärker bevorzugt 40 bis 100 °C und am stärksten bevorzugt 50 bis 90 °C auf. Die genannten Obergrenzen sollten eingehalten werden, um zu gewährleisten, dass die genannte äußere Schale eine geringe Schichtdicke mit einer hohen Konzentration an Edelmetall aufweist.

Als Prozessgas wird vorzugsweise Luft verwendet, es können aber auch inerte Gase wie zum Beispiel Stickstoff, CO₂, Helium, Neon, Argon oder deren Mischungen verwendet werden.

Wie bereits weiter oben erwähnt, wird das Aufsprühen (Beschichten) der Vorläuferverbindungen auf den Katalysatorträgerkörper in den Schritten (b) und (c) und dem optionalen Schritt (c1) vorzugsweise durch Auftragen aus wässrigen Lösungen durchgeführt. Als Lösungsmittel für die Übergangsmetall-Vorläuferverbindungen sind Wasser und Mischungen aus Wasser und Lösungsmitteln, vorzugsweise jedoch entionisiertes Wasser, geeignet, in denen die ausgewählte(n) Metallverbindung(en) löslich ist/sind und die nach dem Auftrag auf den Katalysatorträger von denselben leicht mittels Trocknung wieder entfernt werden können. Bevorzugte Lösungsmittel sind unsubstituierte Carbonsäuren, insbesondere Essigsäure und Ketone, wie Aceton.

Das Aufsprühen der die Vorläuferverbindungen enthaltenden Lösungen wird in allen Verfahrensschritten des erfindungsgemäßen Verfahrens vorzugsweise durch Zerstäuben der Lösung mithilfe einer Sprühdüse bewerkstelligt. Hierbei wird vorzugsweise eine Ringspaltdüse verwendet, die eine Sprühwolke versprüht, deren Symmetrieebene vorzugsweise parallel zur Ebene des Gerätebodens verläuft. Durch den 360°- Umfang der Sprühwolke können die mittig herabfallenden Trägerkörper besonders gleichmäßig mit der Lösung besprüht werden. Dabei ist die Ringspaltdüse, d.h. deren Mündung, vorzugsweise vollständig in der die Umwälzbewegung der Trägerkörper durchführenden Apparatur eingebettet.

Entsprechend einer weiter bevorzugten Ausführungsform des erfindungsgemäßen Verfahrens ist es vorgesehen, dass die Ringspaltdüse mittig im Boden der die Umwälzbewegung der Trägerkörper vornehmenden Apparatur angeordnet ist und die Mündung der Ringspaltdüse in der Apparatur vollständig eingebettet ist. Dadurch wird gewährleistet, dass die freie Weglänge der Tropfen der Sprühwolke bis zum Auftreffen auf einen Formkörper verhältnismäßig kurz ist und entsprechend den Tropfen verhältnismäßig wenig Zeit verbleibt, zu größeren Tropfen zu koaleszieren, was der Ausbildung einer weitgehend einheitlichen Schalendicke entgegenwirken könnte.

Die Zerstäubung der Lösungen erfolgt in dem erfindungsgemäßen Verfahren vorzugsweise so, dass die Lösungen in der Form eines Aerosols auf die Trägerkörper auftreffen. Die Tröpfchengröße liegt hierbei bei dem Auftreffen auf die Trägerkörper vorzugsweise im Bereich von 1 bis 100 µm, bevorzugt von 10 bis 40 µm. Es wurde festgestellt, dass eine zu kleine Tröpfchengröße zu Katalysatoren führt, die eine schlechte Aktivität aufweisen, wohingegen eine zu große Tröpfchengröße zu Katalysatoren mit einer schlechten VAM-Selektivität führt.

Die in Schritt (b) und dem optionalen Schritt (c1) verwendeten die Au-haltige Vorläuferverbindung enthaltenden Lösungen enthalten Au vorzugsweise im Bereich von 0,01 bis 10 Gew.-%, stärker bevorzugt 0,1 bis 8 Gew.-%, noch stärker bevorzugt 0,2 bis 5 Gew.-% und am stärksten bevorzugt 0,3 bis 2 Gew.-%, bezogen auf den atomaren Gew.-Anteil des Metalls in Lösung. Gleiches gilt auch für eine Lösung, die die Au-haltige Vorläuferverbindung enthält, die zeitgleich mit der in Schritt (c) verwendeten Lösung aufgebracht wird.

Die in Schritt (c) verwendete die Pd-haltige Vorläuferverbindung enthaltende Lösung enthält Pd vorzugsweise im Bereich von 0,1 bis 10 Gew-%, stärker bevorzugt 0,5 bis 7 Gew.-%, noch stärker bevorzugt 0,7 bis 5 Gew.-% und am stärksten bevorzugt 1 bis 2,5 Gew.-%, bezogen auf den atomaren Gew.-Anteil des Metalls in Lösung.

Wird in Schritt (c) eine Lösung verwendet, die neben der Pd-haltigen Vorläuferverbindung auch eine Au-haltige Vorläuferverbindung umfasst, so enthält diese Lösung vorzugsweise die gleichen Anteile an Pd und Au wie für die einzelnen Lösungen angegeben.

Liegen bei den zuvor angegebenen Lösungen die Metallgehalte oberhalb der angegebenen Bereiche, dann kommt es zu Schalenkatalysatoren, die eine geringere Selektivität aufweisen. Metallgehalte unterhalb der angegebenen Bereiche wirken sich negativ auf die Aktivität der erhaltenen Katalysatoren aus und führen zu erheblich längeren Aufsprühzeiten mit entsprechender nachteiliger Auswirkung auf die Wirtschaftlichkeit des Verfahrens.

Es ist möglich, dass nach dem Aufsprühen der Vorläuferverbindungen auf den Trägerkörper in Schritt (b) ein Trocknungsschritt erfolgt, bevor in Schritt (c) die Pd-haltige Vorläuferverbindung aufgesprüht wird. Gleiches gilt auch optional, wenn nach Schritt (c) der Schritt (c1) durchgeführt wird. Der Trocknungsschritt wird vorzugsweise unterhalb der Zersetzungstemperatur der Vorläuferverbindungen, insbesondere bei den weiter oben genannten Temperaturen, durchgeführt. Unter einer Zersetzungstemperatur versteht man die Temperatur, bei der sich die Vorläuferverbindungen zu zersetzen beginnen. Das Trocknen erfolgt vorzugsweise entweder durch die Prozessluft im Fließbett oder der Wirbelschicht, durch Stehenlassen an der Luft oder in einem Trocknungsofen, vorzugsweise bei einer Temperatur im Bereich von 60°C bis 120°C. Es ist jedoch weiterhin besonders bevorzugt, dass die Schritte (b), (c) und optional (c1) unmittelbar aufeinander folgen, so dass zwischen diesen kein Trocknungsschritt durchgeführt wird. Dies erfolgt, indem unmittelbar nach dem Aufsprühen der Lösung enthaltend die Vorläuferverbindung in Schritt (b) die Lösung enthaltend die Pd-haltige Vorläuferverbindung aus Schritt (c), bzw. unmittelbar nach Schritt (c) die Lösung enthaltend die Au-haltige Vorläuferverbindung aus dem optionalen Schritt (c1) aufgesprüht wird.

Zudem kann optional zwischen dem Aufsprühen der Lösungen in Schritten (b) und (c) (und optional (c1)) ein Schritt der Zwischenkalzinierung zur Oxidation der Vorläuferverbindung zu den Metalloxiden durchgeführt werden. Ebenso kann optional ein Schritt der Zwischenreduktion zur Reduktion der Vorläuferverbindungen zu den Metallen durchgeführt werden. Besonders bevorzugt ist es jedoch, dass weder ein Schritt der Trocknung noch der Kalzinierung noch der Reduktion zwischen den Schritten des Aufsprühens der beiden Vorläuferverbindungen in Schritten (b), (c) und optional (c1) erfolgt.

Der Katalysatorträgerkörper enthält nach Schritt (b) vorzugsweise einen Anteil an Au im Bereich von 0,1 bis 1,0 Gew.-%,
bezogen auf das Gesamtgewicht des Katalysatorträgerkörpers nach der Trocknung (d.h. bezogen auf den wasserfreien Katalysatorpräkursor) .

Der Katalysatorträgerkörper enthält nach Schritt (c) vorzugsweise einen Anteil an Pd im Bereich von 0,6 bis 2 Gew.-%, stärker bevorzugt im Bereich von 0,7 bis 1,6 Gew.-%, bezogen auf das Gesamtgewicht des Katalysatorträgerkörpers nach der Trocknung. Der Katalysatorträgerkörper enthält nach Schritt (c) vorzugsweise einen Anteil an Au im Bereich von 0,1 bis 1 Gew.-%, stärker bevorzugt im Bereich von 0,3 bis 0,6 Gew.-%, bezogen auf das Gesamtgewicht des Katalysatorträgerkörpers nach der Trocknung.

Die Au-haltige Vorläuferverbindung und Pd-haltige Vorläuferverbindung in den Schritten (b), (c) und optional (c1) ist vorzugsweise eine wasserlösliche Verbindung.

Die Pd-haltige Vorläuferverbindung wird vorzugsweise ausgewählt aus: Nitrat-Verbindungen, Nitrit-Verbindungen, Acetat-Verbindungen, Tetraammin-Verbindungen, Diammin-Verbindungen, Hydrogencarbonat-Verbindungen und hydroxidischen Metallat-Verbindungen.

Beispiele für bevorzugte Pd-haltige Vorläuferverbindungen sind wasserlösliche Pd-Salze. Gemäß einer besonders bevorzugten Ausführungsform des erfindungsgemäßen Verfahrens ist die Pd-Vorläuferverbindung ausgewählt aus der Gruppe bestehend aus Pd(NH₃)₄(HCO₃)₂, Pd(NH₃)₄(HPO₄), Ammonium-Pd-Oxalat, Pd-Oxalat, K₂Pd(Oxalat)₂, Pd(II)-Trifluoracetat, Pd(NH₃)₄(OH)₂, Pd(NO₃)₂, H₂Pd(OAc)₂(OH)₂, Pd(NH₃)₂(NO₂)₂, Pd(NH₃)₄(NO₃)₂, H₂Pd(NO₂)₄, Na₂Pd(NO₂)₄, Pd(OAc)₂ sowie frisch ausgefälltes Pd(OH)₂. Die Herstellung von frisch ausgefällten Pd(OH)₂ wird vorzugsweise folgendermaßen durchgeführt: Hierbei wird vorzugsweise eine 0,1 bis 40 Gew.-% wässrige Lösung aus Tetrachloropalladat hergestellt. Dann wird zu dieser Lösung eine Base, vorzugsweise eine wässrige Lösung aus Kaliumhydroxid hinzugegeben, bis ein brauner Feststoff, nämlich das Pd(OH)₂ ausfällt. Zur Herstellung einer Lösung zum Auftragen auf den Katalysatorträger wird das frisch ausgefällte Pd(OH)₂ isoliert, gewaschen und in einer wässrig alkalischen Lösung gelöst. Das Lösen erfolgt vorzugsweise bei einer Temperatur im Bereich von 4 bis 40 °C, besonders bevorzugt 15 bis 25 °C. Eine tiefere Temperatur ist aufgrund des Gefrierpunktes des Wassers nicht möglich, eine höhere Temperatur bringt den Nachteil mit sich, das Pd(OH)₂ sich in der wässrigen Lösung nach einer bestimmten Zeit wieder niederschlägt und nicht in Lösung geht.

Weiterhin können in dem erfindungsgemäßen Verfahren auch die Pd-Nitrit-Vorläuferverbindungen eingesetzt werden. Bevorzugte Pd-Nitrit-Vorläuferverbindungen sind beispielsweise solche, die mittels Lösen von Pd(OAc)₂ in einer NaNO₂- oder KNO₂-Lösung erhalten werden.

Die Au-haltigen Vorläuferverbindungen in den Schritten (b) und (c) und dem optionalen Schritt (c1) werden jeweils unabhängig voneinander vorzugsweise ausgewählt aus: Acetat-Verbindungen, Nitrit- oder Nitrat-Verbindungen und hydroxidischen Metallatverbindungen.

Beispiele für bevorzugte Au-haltige Vorläuferverbindungen sind wasserlösliche Au-Salze. Gemäß einer besonders bevorzugten Ausführungsform des erfindungsgemäßen Verfahrens ist die Au-Vorläuferverbindung ausgewählt aus der Gruppe bestehend aus KAuO₂, NaAuO₂, LiAuO₂, RbAuO₂, Ba(AuO₂)₂, NaAu(OAc)₃(OH), KAu(NO₂)₄, KAu(OAc)₃(OH), LiAu(OAc)₃(OH), RbAu(OAc)₃(OH), HAu(NO₃)₄ und Au(OAc)₃. Dabei ist es gegebenenfalls empfehlenswert, dass Au(OAc)₃ oder das KAuO₂ mittels Fällung des Oxids, Hydroxids aus einer Goldsäure-Lösung, Waschung und Isolierung des Niederschlags sowie Aufnahme desselben in Essigsäure bzw. KOH jeweils frisch anzusetzen. Besonders bevorzugt wird als Au-haltige Vorläuferverbindung Kaliumaurat eingesetzt, welches in gelöster Form zum Aufbringen auf den Trägerkörper verwendet wird. Die Herstellung einer Kaliumauratlösung ist in der Literatur bekannt und kann nach den in den Druckschriften WO 99/62632 und US 6,015,769 offenbarten Herstellungsverfahren hergestellt werden. Insbesondere bevorzugt sind die Au-haltigen Vorläuferverbindungen in den Schritten (b) und (c) und dem optionalen Schritt (c1) die gleichen, insbesondere KAuO₂.

Die genannten Vorläuferverbindungen werden nur beispielhaft aufgeführt und es können jegliche weitere Vorläuferverbindungen verwendet werden. Besonders bevorzugt ist es, dass die Vorläuferverbindungen im Wesentlichen chlorid-frei sind. Unter im Wesentlichen chlorid-frei versteht man, dass die Summenformel der Verbindung kein Chlorid umfasst, es jedoch nicht ausgeschlossen ist, dass die Verbindung beispielsweise herstellungsbedingt unvermeidliche Verunreinigungen an Chlorid enthält. Hierbei ist es besonders bevorzugt, dass der maximale Chloridgehalt in einer Lösung enthaltend die Au-Vorläuferverbindung nicht über 5000 ppm, stärker bevorzugt über 3000 ppm und am stärksten bevorzugt nicht über 1500 ppm liegt, und in einer Lösung enthaltend die Pd-Vorläuferverbindung nicht über 600 ppm, stärker bevorzugt über 300 ppm und am stärksten bevorzugt nicht über 100 ppm liegt.

Insbesondere bevorzugt werden in dem erfindungsgemäßen Verfahren als Pd-haltige
keinen oder nahezu keinen Sauerstoff oder andere oxidierend wirkende Gase enthält. Die nicht-oxidierende Atmosphäre kann eine Atmosphäre aus Inertgas oder eine reduzierende Atmosphäre oder eine Mischung aus beiden Gasvarianten sein.

In einer Variante des erfindungsgemäßen Verfahrens wird die Reduktion in einer Atmosphäre aus Inertgas durchgeführt. In diesem Fall wirken die Gegenionen des Metallions in der metallhaltigen Vorläuferverbindung reduzierend oder die Metallkomplexe disproportionieren unter den gewählten Prozessbedingungen zur Oxidationsstufe null.

In einer weiteren Variante des erfindungsgemäßen Verfahrens kann die Temperaturbehandlung direkt in einer Vorläuferverbindung Pd(NH₃)₄(OH)₂ und als Au-haltige Vorläuferverbindung NaAuO₂ oder KAuO₂, besonders bevorzugt KAuO₂, eingesetzt.

Nach den Schritten des Aufsprühens der Vorläuferverbindungen und vor dem Schritt (d) wird der Trägerkörper vorzugsweise einem Trocknungsschritt unterzogen. Der Trocknungsschritt erfolgt vorzugsweise auf die gleiche Weise wie weiter oben in Verbindung mit den optionalen Trocknungsschritten zwischen den Schritten (b) und (c) bzw. (c1).

Wie bereits ausgeführt wird der Schritt (d) vorzugsweise in einer nicht-oxidierenden Atmosphäre zur Reduktion der Metallkomponenten der Vorläuferverbindung zu den elementaren Metallen durchgeführt.

Die Temperaturbehandlung in einer nicht-oxidierenden Atmosphäre wird vorzugsweise in einem Temperaturbereich von 60°C bis 500°C.

Unter einer nicht-oxidierenden Atmosphäre versteht man in der vorliegenden Erfindung eine Atmosphäre, die reduzierenden Atmosphäre durchgeführt werden. In diesem Fall werden die Vorläuferverbindungen gleichzeitig zersetzt und die Metallkomponente zu den elementaren Metallen reduziert. D.h. Zersetzung und Reduktion werden gleichzeitig bei der gleichen Temperatur in der reduzierenden Atmosphäre durchgeführt. In diesem Fall findet die Temperaturbehandlung vorzugsweise in einem Bereich von größer oder gleich 40 °C bis 400 °C, stärker bevorzugt 50 °C bis 300 °C, noch stärker bevorzugt 60 °C bis 250 °C und am stärksten bevorzugt im Bereich von 70 °C bis 180 °C statt.

In einer noch weiteren Variante des erfindungsgemäßen Verfahrens wird die Temperaturbehandlung vorzugsweise so durchgeführt, dass während der Temperaturbehandlung eine Umstellung von einer Atmosphäre aus Inertgas auf eine reduzierende Atmosphäre erfolgt. Somit ist dieses Verfahren Energie- und Kosten-sparend. Dies ist insbesondere möglich, da von Ausgangsverbindungen ausgegangen wird, die keine so hohen Zersetzungstemperaturen aufweisen, wie beispielsweise chlorhaltige Verbindungen.

Hierbei werden die Vorläuferverbindungen zunächst in einer Atmosphäre aus Inertgas bei ihrer Zersetzungstemperatur zersetzt und anschließend durch die Umstellung auf eine reduzierende Atmosphäre die Metallkomponenten zu den elementaren Metallen reduziert. Die Temperatur während der Zersetzung unter Inertgas liegt vorzugsweise im Bereich von 200 bis 500 °C, stärker bevorzugt 250 bis 450 °C und am stärksten bevorzugt über 300 °C. Die Temperatur während der anschließenden Reduktion liegt dann vorzugsweise im Bereich von größer oder gleich 40 °C bis 400 °C, stärker bevorzugt 40 °C bis 300 °C, noch stärker bevorzugt 45 °C bis 250 °C und am stärksten bevorzugt im Bereich von 50 °C bis 180 °C.

Alle drei Verfahrensvarianten haben den Vorteil, dass auf eine Vorkalzinierung bzw. Zwischenkalzinierung in einem weiteren vorgeschalteten oder zwischengeschalteten Schritt in einer anderen Anlage verzichtet werden kann. Somit wird das erfindungsgemäße Verfahren vorzugsweise durchgeführt, indem auf aufwendiges Abkühlen auf unterhalb der Zersetzungstemperatur und Aufheizen auf oberhalb der Zersetzungstemperatur verzichtet wird.

Erfindungsgemäß ist es besonders bevorzugt, dass die Umstellung von einer Atmosphäre aus Inertgas auf eine reduzierende Atmosphäre so durchgeführt wird, dass die Temperatur während des Umstellens nicht unter die für die Reduktion erwünschte Temperatur absinkt.

Als Inertgas werden beispielsweise N₂, He, Ne, Ar oder Mischungen davon verwendet. Besonders bevorzugt wird N₂ verwendet.

Die reduktiv wirkende Komponente in der reduzierenden Atmosphäre ist in der Regel in Abhängigkeit von der Natur der zu reduzierenden Metallkomponente auszuwählen, jedoch vorzugsweise ausgewählt aus der Gruppe von Gasen oder verdampfbaren Flüssigkeiten bestehend aus Ethylen, Wasserstoff, CO, NH₃, Formaldehyd, Methanol, Ameisensäure und Kohlenwasserstoffen, oder ist eine Mischung von zwei oder mehr der vorgenannten Gase/Flüssigkeiten. Besonders bevorzugt umfasst die reduzierende Atmosphäre Wasserstoff als reduzierende Komponente. Insbesondere bevorzugt ist es, wenn die reduzierende Atmosphäre aus Formiergas, einem Gemisch aus N₂ und H₂, gebildet wird. Der Wasserstoffgehalt befindet sich hierbei im Bereich von 1 Vol.-% bis 15 Vol.-%. Das erfindungsgemäße Verfahren wird beispielsweise mit Wasserstoff (4 - 5 Vol.-%) in Stickstoff als Prozessgas bei einer Temperatur im Bereich von 60 °C bis 500 °C über einen Zeitraum von beispielsweise 1 bis 5 Stunden durchgeführt.

Das in der zweiten Verfahrensalternative genannte Umstellen von Inertgas auf eine reduzierende Atmosphäre erfolgt vorzugsweise, indem in eine Atmosphäre aus Inertgas eine der genannten reduzierenden Komponenten zugeführt wird. Vorzugsweise wird hierbei Wasserstoffgas zugeführt. Die Zuführung eines reduzierend wirkenden Gases zu dem Inertgas hat den Vorteil, dass die Temperatur nicht wesentlich absinkt bzw. nicht bis bzw. unter die für die Reduktion erwünschte Untergrenze von mindestens 60 °C absinkt, sodass kein erneutes Kosten- und Energie-intensives Aufheizen durch einen entsprechenden totalen Atmosphärenaustausch notwendig ist.

In einer besonders bevorzugten Ausführungsform wird der die Vorläuferverbindungen enthaltende Trägerkörper vor der Temperaturbehandlung nicht einer Temperatur von größer oder gleich 300 °C in einer oxidierenden Atmosphäre ausgesetzt. Auf diese Weise ist gewährleistet, dass der Trägerkörper mit den darauf aufgebrachten Vorläuferverbindungen, so wie die Vorläuferverbindungen sind, der Temperaturbehandlung unterzogen wird. In anderen Worten: es kann auf eine kostenintensive Vorkalzinierung des imprägnierten Trägerkörpers zu den Metalloxiden verzichtet werden. Es ist aber erfindungsgemäß auch möglich, dass eine Zwischenkalzinierung zu Oxiden durchgeführt wird.

Der mit dem erfindungsgemäßen Verfahren hergestellte Schalenkatalysator enthält einen Gesamtanteil an Au im Bereich von 0,1 bis 1,0 Gew.-%, bevorzugt 0,2 bis 0,9 Gew.-%, stärker bevorzugt 0,25 bis 0,8 Gew.-% und am stärksten bevorzugt im Bereich von 0,3 bis 0,7 Gew.-% bezogen auf das Gesamtgewicht des Schalenkatalysators.

Der mit dem erfindungsgemäßen Verfahren hergestellte Schalenkatalysator enthält einen Gesamtanteil an Pd im Bereich von 0,6 bis 2,0 Gew.-%, bevorzugt 0,8 bis 1,7 Gew.-%, stärker bevorzugt 0,9 bis 1,6 Gew.-% und am stärksten bevorzugt im Bereich von 1,0 bis 1,5 Gew.-% bezogen auf das Gesamtgewicht des Schalenkatalysators.

Nach der Reduktion der Metallkomponenten der Vorläuferverbindungen zu den elementaren Metallen wird in dem erfindungsgemäßen Verfahren vorzugsweise der Promoter KOAc auf den nachvergoldeten Träger aufgebracht, indem der erhaltene Katalysatorpräkursor mit einer wässrigen KOAc-Lösung (vorzugsweise wässrige Lösung) nach der Porenfüllmethode (incipient wetness) bei Raumtemperatur imprägniert wird und typischerweise etwa eine Stunde stehengelassen wird, bevor die Trocknung eingeleitet wird. Die Kaliumbeladung liegt vorzugsweise im Bereich von 2 bis 3,5 Gew.-%, stärker bevorzugt 2,2 bis 3,0 Gew.-% und am bevorzugtesten 2,5 bis 2,7 Gew.-% bezogen auf das Gesamtgewicht des trockenen Katalysators. Nach dem Aufbringen der KOAc-Lösung kann eine finale Trocknung im Bereich von 70-120°C, stärker bevorzugt 80-110°C und am stärksten bevorzugt 90-100°C in Luft, Magerluft oder Inertgas erfolgen.

Ein weiterer Gegenstand der vorliegenden Erfindung ist zudem ein Schalenkatalysator, der nach dem erfindungsgemäßen Verfahren erhältlich ist. Der erfindungsgemäße Schalenkatalysator unterscheidet sich von herkömmlichen Schalenkatalysatoren für die Synthese von Vinylacetat und/oder Allylacetat dadurch, dass er eine signifikant höhere Selektivität und Aktivität bei der Synthese von Vinylacetat und/oder Allylacetat aufweist. Die anhand der besseren Selektivität und Aktivität des erfindungsgemäßen Schalenkatalysators im Vergleich zu herkömmlichen Katalysatoren eindeutig vorliegenden strukturellen Unterschiede lassen sich zum Zeitpunkt der Anmeldung nicht in physikalische Größen fassen. Deshalb kann der erfindungsgemäße Schalenkatalysator nur durch die Art seiner Herstellung und die festgestellte erhöhte Selektivität und Aktivität von herkömmlichen Katalysatoren unterschieden werden.

Eine weitere Ausführungsform bezieht sich auf die Verwendung eines nach einem erfindungsgemäßen Verfahren hergestellten Schalenkatalysators Oxy-acetylierung von Olefinen, insbesondere zur Herstellung von Allylacetat oder Vinylacetat. In anderen Worten betrifft die vorliegende Erfindung auch ein Verfahren zur Oxy-acetylierung von Olefinen, in dem Essigsäure, ein Olefin und Sauerstoff oder Sauerstoff enthaltende Gase über den erfindungsgemäßen Katalysator geleitet werden. Olefin ist hier bevorzugt Ethylen oder Propylen. Das Verfahren erfolgt im Allgemeinen durch Leiten von Essigsäure, Olefin und Sauerstoff oder Sauerstoff enthaltenden Gasen bei Temperaturen von 100-200 °C, vorzugsweise 120-200 °C, und bei Drücken von 1-25 bar, vorzugsweise 1-20 bar, über den erfindungsgemäßen Katalysator, wobei nicht umgesetzte Edukte im Kreis geführt werden können. Zweckmäßig hält man die Sauerstoffkonzentration unter 10 Vol.-%. Unter Umständen ist jedoch auch eine Verdünnung mit inerten Gasen wie Stickstoff oder Kohlendioxid vorteilhaft. Besonders Kohlendioxid eignet sich zur Verdünnung, da es im Zuge der Vinylacetat- und/oder Allylacetat-Synthese in geringen Mengen gebildet wird und sich im Kreisgas ansammelt. Das entstandene Vinylacetat/Allylacetat wird mit Hilfe geeigneter Methoden isoliert, die beispielsweise in der US 5,066,365 A beschrieben sind.

Die Erfindung ist nachstehend anhand einer Figur und von Ausführungsbeispielen näher erklärt, ohne dass diese als einschränkend verstanden werden sollen.

Figur:

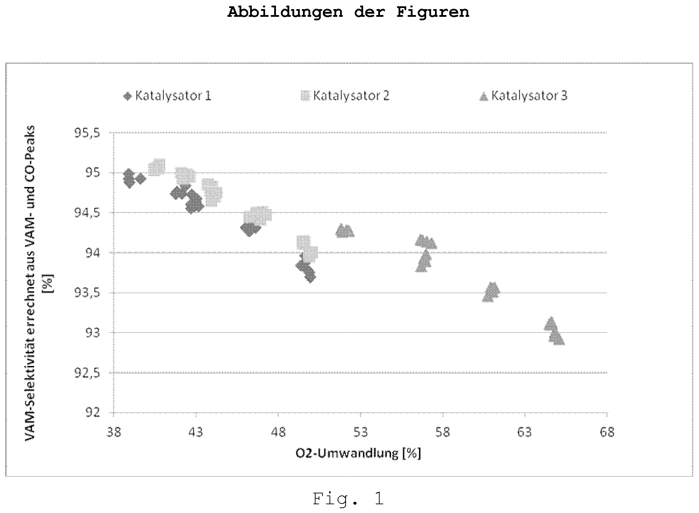

- Figur 1:: Fig. 1 zeigt die VAM-Selektivitäten errechnet aus den gemessenen VAM- und CO₂-Peaks in Abhängigkeit von der O₂-Umwandlung unter Verwendung von 3 verschiedenen Schalenkatalysatoren bei der katalytischen Synthese von VAM.

### Beispiele:

Bei den Prozentabgaben bezüglich der Lösungen enthaltend die Vorläuferverbindungen handelt es sich um atomare Gew.-% des jeweiligen Metalls bezogen auf das Gesamtgewicht der Lösung.

### Vergleichsbeispiel 1: Katalysator 1:

100g des Trägers "KA-Zr-14" (mit 14% ZrO₂ dotierter KA-160 Träger der Süd-Chemie) wird mit einer wässrigen Mischlösung aus Pd(NH₃)₄(OH)₂ und KAuO₂ (hergestellt durch Mischen von 27,79g einer 4,70%-igen Pd-Lösung + 12,09g einer 3,60%-igen Au-Lösung + 50ml Wasser) in einem Innojet IAC025 Coater bei 70°C beschichtet (Sprührate 80%, d.h. es wurden 4 g Lösung pro Minute pro 100 g Träger aufgetragen), anschließend bei 90°C für 40min in einem Wirbelschichttrockner getrocknet und bei 150°C für 4h mit Formiergas reduziert. Nach der Reduktion wird eine 2-molare KOAc-Lösung mit dem "incipient wetness Prinzip" imprägniert, die Imprägnierzeit beträgt eine Stunde. Anschließend wird erneut in einem Wirbelschichttrockner bei 90°C für 40min getrocknet. Die durch chemische Elementanalytik bestimmten LOI-freien (LOI: loss of ignition) Metallgehalte des fertigen Katalysators betragen 1,15% Pd + 0,41% Au.

### Vergleichsbeispiel 2: Katalysator 2:

100g des Trägers "KA-Zr-14" (mit 14% ZrO₂ dotierter KA-160 Träger der Süd-Chemie) wird mit einer wässrigen Lösung aus 27,79g Pd(NH₃)₄(OH)₂ (hergestellt durch Mischen von 27,79g einer 4,70%-igen Pd-Lösung + 50ml Wasser) (4,70%) in einem Innojet IAC025 Coater bei 70°C beschichtet (Sprührate 80%, d.h. es wurden 4 g Lösung pro Minute pro 100 g Träger aufgetragen) und anschließend 12,09g KAuO₂ (hergestellt durch Mischen von 12,09g einer 3,60%-igen Au-Lösung + 50ml Wasser) in einem Innojet IAC025 Coater bei 70°C beschichtet (Sprührate 80%, d.h. es wurden 4 g Lösung pro Minute pro 100 g Träger aufgetragen), bei 90°C für 40min in einem Wirbelschichttrockner getrocknet und bei 150°C für 4h mit Formiergas reduziert. Nach der Reduktion wird eine 2-molare KOAc-Lösung mit dem "incipient wetness"-Prinzip imprägniert. Die Imprägnierzeit beträgt eine Stunde. Anschließend wird erneut in einem Wirbelschichttrockner bei 90°C für 40min getrocknet. Die durch chemische Elementanalytik bestimmten LOI-freien Metallgehalte des fertigen Katalysators betragen 1,16% Pd + 0,41% Au.

### Beispiel 1: Katalysator 3:

100g des Trägers "KA-Zr-14" (mit 14% ZrO₂ dotierter KA-160 Träger der Süd-Chemie) wird mit einer wässrigen Lösung aus 12,09g KAuO₂ (hergestellt durch Mischen von 12,09g einer 3,60%-igen Au-Lösung + 50ml Wasser)in einem Innojet IAC025 Coater bei 70°C beschichtet (Sprührate 80%, d.h. es wurden 4 g Lösung pro Minute pro 100 g Träger aufgetragen) und anschließend 27,79g Pd(NH₃)₄(OH)₂ (hergestellt durch Mischen von 27,79g einer 4,70%-igen Pd Lösung + 50ml Wasser)in einem Innojet IAC025 Coater bei 70°C beschichtet (Sprührate 80%, d.h. es wurden 4 g Lösung pro Minute pro 100 g Träger aufgetragen), bei 90°C für 40min in einem Wirbelschichttrockner getrocknet und bei 150°C für 4h mit Formiergas reduziert. Nach der Reduktion wird eine 2-molare KOAc-Lösung mit dem "incipient wetness"-Prinzip imprägniert. Die Imprägnierzeit beträgt eine Stunde. Anschließend wird erneut in einem Wirbelschichttrockner bei 90°C für 40min getrocknet. Die durch chemische Elementanalytik bestimmten LOI-freien Metallgehalte des fertigen Katalysators betragen 1,17% Pd + 0,41% Au.

### Beispiel 2: Katalysator 4:

100g des Trägers "KA-Zr-14" (mit 14% ZrO₂ dotierter KA-160 Träger der Süd-Chemie) wird mit einer wässrigen Lösung aus 3,02g KAuO₂ (hergestellt durch Mischen von 3,02g einer 3,60%-igen Au-Lösung + 50ml Wasser) in einer Dragiertrommel, (Drageekessel DF-LDP-3 der Fa. D&F Drouven GmbH, siehe auch beispielhaft DE 10 2005 061382 A1)bei 50°C beschichtet (Sprührate 80%, d.h. es wurden 4 g Lösung pro Minute pro 100 g Träger aufgetragen) und anschließend mit einer Mischlösung aus 27,79g Pd(NH₃)₄(OH)₂ und 9,07g KAuO₂ (hergestellt durch Mischen von 27,79g einer 4,70%-igen Pd Lösung + 9,07g einer 3,60%-igen Au-Lösung + 50ml Wasser) in einer Dragiertrommel bei 50°C beschichtet (Sprührate 80%, d.h. es wurden 4 g Lösung pro Minute pro 100 g Träger aufgetragen), bei 90°C für 40min in einem Wirbelschichttrockner getrocknet und bei 150°C für 4h mit Formiergas reduziert. Nach der Reduktion wird eine 2-molare KOAc-Lösung mit dem "incipient wetness"-Prinzip imprägniert, die Imprägnierzeit beträgt eine Stunde. Anschließend wird erneut in einem Wirbelschichttrockner bei 90°C für 40min getrocknet. Die durch chemische Elementanalytik bestimmten LOI-freien Metallgehalte des fertigen Katalysators betragen 1,19% Pd + 0,42% Au.

### Beispiel 3: Testergebnisse

der Katalysatoren 1 bis 3 hinsichtlich ihrer Selektivität bei der Synthese von Vinylacetatmonomer:
Dazu wurden Essigsäure, Ethylen und Sauerstoff bei einer Temperatur von 140°C/12 h --> 143°C/12 h --> 146°C/12 h (es handelt es sich um die jeweiligen Reaktionstemperaturen, die nach der Reihe bei der automatisierten Abarbeitung des Screening-Protokolls angefahren werden, d.h. es wird 12 h lang bei 140°C gemessen, danach 12 h lang bei 143°C, und danach 12 h lang bei 146°C Reaktortemperatur gemessen) und einem Druck von 6,5 bar, jeweils über die Katalysatoren 1 bis 3 geleitet. Die Konzentrationen der verwendeten Komponenten betrugen: 40,2% Ethylen, 6,3% O₂, 0,6% CO₂, 9,6% Methan, 12,68% Essigsäure, Rest N₂.

Die Testergebnisse für die Katalysatoren 1 bis 3 können der Figur 1 und der folgenden Tabelle 1 entnommen werden. Der Katalysator 4 zeigt bei vergleichbaren Verfahrensbedingungen eine Selektivität, die zwischen der Selektivität der Katalysatoren 1 und 2 und der Selektivität des Katalysators 3 liegt.

**Tabelle 1:**

| Katalysator 1 | | Katalysator 2 | | Katalysator 3 | |
|---|---|---|---|---|---|
| Selektivität errechnet aus VAM- und CO2-Peaks [%] | O2-Umwandlung [%] | Selektivität errechnet aus VAM- und CO2-Peaks [%] | O2-Umwandlung [%] | Selektivität errechnet aus VAM- und CO2-Peaks [%] | O2-Umwandlung [%] |
| 94,98 | 38,88 | 95,06 | 40,73 | 94,29 | 51,86 |
| 94,92 | 38,92 | 95,07 | 40,66 | 94,26 | 51,88 |
| 94,92 | 38,91 | 95,1 | 40,75 | 94,3 | 51,84 |
| 94,88 | 38,95 | 95,04 | 40,46 | 94,28 | 52,15 |
| 94,92 | 39,64 | 95,02 | 40,47 | 94,27 | 52,28 |
| 94,55 | 42,71 | 94,7 | 44,16 | 93,83 | 56,68 |
| 94,58 | 42,9 | 94,65 | 43,95 | 93,89 | 56,95 |
| 94,58 | 43,16 | 94,74 | 44,21 | 93,93 | 56,86 |
| 94,63 | 42,98 | 94,77 | 43,88 | 93,98 | 56,99 |
| 94,6 | 42,67 | 94,81 | 43,87 | 93,92 | 56,86 |
| 94,64 | 42,94 | 94,82 | 43,97 | 93,92 | 56,91 |
| 94,68 | 43,01 | 94,85 | 43,7 | 93,46 | 60,73 |
| 94,28 | 46,29 | 94,47 | 47,26 | 93,51 | 61,02 |
| 94,31 | 46,6 | 94,41 | 46,92 | 93,56 | 61,03 |
| 94,33 | 46,51 | 94,51 | 47,02 | 93,54 | 60,94 |
| 94,32 | 46,3 | 94,49 | 46,87 | 93,56 | 60,9 |
| 94,27 | 46,25 | 94,49 | 46,68 | 93,56 | 61,15 |
| 94,31 | 46,02 | 94,44 | 46,24 | 93,55 | 60,98 |
| 94,29 | 46,42 | 94 | 49,92 | 93,01 | 64,82 |
| 93,75 | 49,91 | 94,01 | 50,08 | 92,92 | 65,06 |
| 93,77 | 49,82 | 93,95 | 49,89 | 92,96 | 64,86 |
| 93,69 | 49,95 | 93,98 | 49,88 | 92,97 | 64,77 |
| 93,96 | 49,66 | 94,11 | 49,53 | 93,11 | 64,67 |
| 93,84 | 49,37 | 94,13 | 49,6 | 93,13 | 64,61 |
| 93,89 | 49,66 | 94,14 | 49,49 | 93,10 | 64,49 |
| 94,73 | 42,76 | 94,95 | 42,62 | 94,12 | 57,3 |
| 94,84 | 42,34 | 94,97 | 42,44 | 94,14 | 57,02 |
| 94,73 | 42,15 | 94,95 | 42,22 | 94,16 | 56,78 |
| 94,73 | 41,76 | 94,92 | 42,17 | 94,16 | 56,62 |
| 94,76 | 41,84 | 94,99 | 42,1 | | |

## Patentansprüche

1. Verfahren zur Herstellung eines Schalenkatalysators umfassend die folgenden Schritte:
(a) Unterziehen einer Schüttung eines Katalysatorträgerkörpers einer Umwälzbewegung;
(b) In-Kontaktbringen einer zerstäubten wässrigen Lösung enthaltend eine Au-haltige Vorläuferverbindung mit der der Umwälzbewegung unterzogenen Schüttung des Katalysatorträgerkörpers durch Aufsprühen der Lösung;
(c) In-Kontaktbringen einer zerstäubten wässrigen Lösung enthaltend eine Pd-haltige Vorläuferverbindung mit dem nach Schritt (b) hergestellten Katalysatorträgerkörper; und
(d) Reduktion der Metallkomponenten der Vorläuferverbindungen zu den elementaren Metallen durch Unterziehen des in Schritt (c) erhaltenen Katalysatorträgerkörpers einer Temperaturbehandlung in einer nicht-oxidierenden Atmosphäre,
worin der Schalenkatalysator einen Anteil an Au im Bereich von 0,1 bis 1,0 Gew.-%, bezogen auf das Gesamtgewicht des Schalenkatalysators, und einen Anteil an Pd im Bereich von 0,6 bis 2,0 Gew.-%, bezogen auf das Gesamtgewicht des Schalenkatalysators, aufweist.

2. Verfahren nach Anspruch 1, worin bei dem In-Kontaktbringen in Schritt (c) die Lösung auf eine einer Umwälzbewegung unterzogenen Schüttung des Katalysatorträgerkörpers aufgesprüht wird.

3. Verfahren nach Anspruch 1 oder 2, worin die Temperaturbehandlung in einem Bereich von 60 °C bis 500 °C durchgeführt wird.

4. Verfahren nach einem der Ansprüche 1 bis 3, worin die Umwälzbewegung der Katalysatorträgerkörper mithilfe eines Prozessgases durchgeführt wird.

5. Verfahren nach einem der Ansprüche 1 bis 4, worin die Umwälzbewegung der Katalysatorträgerkörper in einem Fließbett oder einer Wirbelschicht stattfindet.

6. Verfahren nach einem der Ansprüche 1 bis 5, worin das Verhältnis des Gewichts der in Schritt (b) oder (c) aufgesprühten Lösung zu dem Gewicht der Schüttung des Trägerkörpers im Bereich von 0,005 bis 0,1 liegt.

7. Verfahren nach einem der Ansprüche 1 bis 6, worin in Schritt (c) zusätzlich eine Au-haltige Vorläuferverbindung auf den Katalysatorträgerkörper aufgebracht wird.

8. Verfahren nach einem der Ansprüche 1 bis 7, worin zwischen den Schritten (c) und (d) in einem Schritt (c1) eine Au-haltige Vorläuferverbindung auf den in Schritt (c) erhaltenen Katalysatorträgerkörper aufgebracht wird.

9. Verfahren nach einem der Ansprüche 1 bis 8, worin die Pd-haltige Vorläuferverbindung eine Verbindung aus der folgenden Gruppe ist: Nitrat-Verbindung, Nitritverbindung, Acetat-Verbindung, Tetraammin-Verbindung, Diammin-Verbindung, Hydrogencarbonat-Verbindung, hydroxidische Metallat-Verbindungen und Mischungen davon.

10. Verfahren nach einem der Ansprüche 1 bis 9, worin die Au-haltige(n) Vorläuferverbindung(en) aus der folgenden Gruppe ausgewählt ist/sind: Acetat-Verbindung, Nitrit- oder Nitrat-Verbindung und hydroxidische Metallatverbindung.

11. Verfahren nach einem der Ansprüche 1 bis 10, worin die Au-haltige(n) Vorläuferverbindung(en) aus NaAuO₂, KAuO₂, LiAuO₂ und RbAuO₂ ausgewählt ist/sind.

12. Verfahren nach einem der Ansprüche 1 bis 11, worin der Katalysatorträgerkörper nach Schritt (b) einen Anteil an Au im Bereich von 0,1 bis 1,0 Gew.-% aufweist, bezogen auf das Gesamtgewicht des Katalysatorträgerkörpers.

13. Verfahren nach einem der Ansprüche 1 bis 12, worin der Katalysatorträgerkörper nach Schritt (c) einen Anteil an Pd im Bereich von 0,6 bis 2,0 Gew.-% aufweist, bezogen auf das Gesamtgewicht des Katalysatorträgerkörpers.

14. Verfahren nach einem der Ansprüche 1 bis 13, worin die nicht-oxidierende Atmosphäre ein Reduktionsmittel enthält.

15. Verfahren nach Anspruch 14, worin das Reduktionsmittel Wasserstoff ist.

16. Schalenkatalysator, erhältlich nach einem Verfahren gemäß einem der Ansprüche 1 bis 15.

17. Verwendung eines Schalenkatalysators gemäß Anspruch 16 zur Oxy-acetylierung von Olefinen.

## Claims

1. Process for producing an shell catalyst, comprising the following steps:
(a) subjecting a bed of a catalyst support body to a circulating motion;
(b) bringing an atomized aqueous solution comprising an Au-containing precursor compound in contact with the bed of the catalyst support body subjected to the circulating motion by spraying on of the solution;
(c) bringing an atomized aqueous solution comprising a Pd-containing precursor compound in contact with the catalyst support body produced according to step (b); and
(d) reduction of the metal components of the precursor compounds to the elemental metals by subjecting the catalyst support body obtained in step (c) to a temperature treatment in a nonoxidizing atmosphere,
in which the shell catalyst has an Au content in the range from 0.1 to 1.0% by weight, based on the total weight of the shell catalyst, and a Pd content in the range from 0.6 to 2.0% by weight, based on the total weight of the shell catalyst.

2. Process according to Claim 1, in which during the bringing in contact in step (c) the solution is sprayed onto a bed of the catalyst support body subjected to a circulating motion.

3. Process according to Claim 1 or 2, in which the temperature treatment is conducted within a range from 60°C to 500°C.

4. Process according to any of Claims 1 to 3, in which the circulating motion of the catalyst support bodies is conducted with the aid of a process gas.

5. Process according to any of Claims 1 to 4, in which the circulating motion of the catalyst support bodies takes place in a moving bed or fluidized bed.

6. Process according to any of Claims 1 to 5, in which the ratio of the weight of the solution sprayed on in step (b) or (c) to the weight of the bed of the support body is within the range from 0.005 to 0.1.

7. Process according to any of Claims 1 to 6, in which, in step (c), an Au-containing precursor compound is additionally applied to the catalyst support body.

8. Process according to any of Claims 1 to 7, in which, between steps (c) and (d), in a step (c1), an Au-containing precursor compound is applied to the catalyst support body obtained in step (c).

9. Process according to any of Claims 1 to 8, in which the Pd-containing precursor compound is a compound from the following group: nitrate compound, nitrite compound, acetate compound, tetraammine compound, diammine compound, hydrogencarbonate compound, hydroxidic metalate compounds and mixtures thereof.

10. Process according to any of Claims 1 to 9, in which the Au-containing precursor compound(s) is/are selected from the following group: acetate compound, nitrite or nitrate compound and hydroxidic metalate compound.

11. Process according to any of Claims 1 to 10, in which the Au-containing precursor compound(s) is/are selected from NaAuO₂, KAuO₂, LiAuO₂ and RbAuO₂.

12. Process according to any of the Claims 1 to 11, in which the catalyst support body after step (b) has an Au content in the range from 0.1 to 1.0% by weight, based on the total weight of the catalyst support body.

13. Process according to any of Claims 1 to 12, in which the catalyst support body after step (c) has a Pd content in the range from 0.6 to 2.0% by weight, based on the total weight of the catalyst support body.

14. Process according to any of Claims 1 to 13, in which the nonoxidizing atmosphere comprises a reducing agent.

15. Process according to Claim 14, in which the reducing agent is hydrogen.

16. Shell catalyst obtainable by a process according to any of Claims 1 to 15.

17. Use of a shell catalyst according to Claim 16 for the oxyacetylation of olefins.

## Revendications

1. Procédé pour la préparation d'un catalyseur enrobé, comprenant les étapes suivantes :
(a) soumission d'une charge d'un corps support de catalyseur à un mouvement de circulation ;
(b) mise en contact d'une solution aqueuse vaporisée contenant un composé précurseur contenant Au avec la charge soumise au mouvement de circulation du corps support de catalyseur par pulvérisation de la solution ;
(c) mise en contact d'une solution aqueuse vaporisée contenant un composé précurseur contenant Pd avec le corps support de catalyseur préparé selon l'étape (b) ; et
(d) réduction des composants métalliques des composés précurseurs en métaux élémentaires par soumission du corps support de catalyseur obtenu dans l'étape (c) à un traitement thermique dans une atmosphère non oxydante,
dans lequel le catalyseur enrobé présente une proportion de Au dans la plage de 0,1 à 1,0% en poids, par rapport au poids total du catalyseur enrobé, et une proportion de Pd dans la plage de 0,6 à 2,0% en poids, par rapport au poids total du catalyseur enrobé.

2. Procédé selon la revendication 1, dans lequel, lors de la mise en contact dans l'étape (c), la solution est pulvérisée sur une charge du corps support de catalyseur soumise à un mouvement de circulation.

3. Procédé selon la revendication 1 ou 2, dans lequel le traitement thermique est effectué dans une plage de 60°C à 500°C.

4. Procédé selon l'une quelconque des revendications 1 à 3, dans lequel le mouvement de circulation du corps support de catalyseur est effectué à l'aide d'un gaz de procédé.

5. Procédé selon l'une quelconque des revendications 1 à 4, dans lequel le mouvement de circulation du corps support de catalyseur a lieu dans un lit fluidisé ou dans un lit tourbillonnant.

6. Procédé selon l'une quelconque des revendications 1 à 5, dans lequel le rapport du poids de la solution pulvérisée dans l'étape (b) ou (c) au poids de la charge du corps support se situe dans la plage de 0,005 à 0,1.

7. Procédé selon l'une quelconque des revendications 1 à 6, dans lequel, dans l'étape (c), un composé précurseur contenant Au est en outre appliqué sur le corps support de catalyseur.

8. Procédé selon l'une quelconque des revendications 1 à 7, dans lequel, entre les étapes (c) et (d), dans une étape (c1), un composé précurseur contenant Au est appliqué sur le corps support de catalyseur obtenu dans l'étape (c).

9. Procédé selon l'une quelconque des revendications 1 à 8, dans lequel le composé précurseur contenant Pd est un composé du groupe suivant : composé de type nitrate, composé de type nitrite, composé de type acétate, composé de type tétraamine, composé de type diamine, composé de type hydrogénocarbonate, composés de type métallate hydroxyde et leurs mélanges.

10. Procédé selon l'une quelconque des revendications 1 à 9, dans lequel le(s) composé(s) précurseur(s) contenant Au est/sont choisi(s) dans le groupe suivant : composé de type acétate, composé de type nitrite ou nitrate et composé de type métallate hydroxyde.

11. Procédé selon l'une quelconque des revendications 1 à 10, dans lequel le(s) composé(s) précurseur(s) contenant Au est/sont choisi(s) parmi NaAuO₂, KAuO₂, LiAuO₂ et RbAuO₂.

12. Procédé selon l'une quelconque des revendications 1 à 11, dans lequel le corps support de catalyseur présente, après l'étape (b), une proportion de Au dans la plage de 0,1 à 1,0% en poids, par rapport au poids total du corps support de catalyseur.

13. Procédé selon l'une quelconque des revendications 1 à 12, dans lequel le corps support de catalyseur présente, après l'étape (c), une proportion de Pd dans la plage de 0,6 à 2,0% en poids, par rapport au poids total du corps support de catalyseur.

14. Procédé selon l'une quelconque des revendications 1 à 13, dans lequel l'atmosphère non oxydante contient un agent de réduction.

15. Procédé selon la revendication 14, dans lequel l'agent de réduction est l'hydrogène.

16. Catalyseur enrobé pouvant être obtenu selon un procédé selon l'une quelconque des revendications 1 à 15.

17. Utilisation d'un catalyseur enrobé selon la revendication 16 pour l'oxyacétylation d'oléfines.
